# EUROPEAN PATENT APPLICATION

(11) **EP 4 776 302 A2**
(43) Date of publication of application: **15.07.2026**
(21) Application number: 23956869.4
(22) Date of filing: 23.10.2023
(51) Int. Cl.: G16H 50/20, G16H 10/20, G16H 10/60, G16H 70/00, G16H 40/20, G06Q 10/02, G10L 15/02

(54) **ARTIFICIAL INTELLIGENCE DEVICE AND OPERATION METHOD THEREOF**

(71) Applicant: LG Electronics Inc., Yeongdeungpo-gu Seoul 07336 (KR)
(72) Inventor: LEE, Jae Kyung, Seoul 06772 (KR)
(74) Representative: Schott, Jakob Valentin
(86) International application number: PCT/KR2023/016477
(87) International publication number: WO 2025/089433

(57) **Abstract**

An artificial intelligence device according to an embodiment of the present disclosure receives a voice command uttered by a user, obtains an analysis result of the received voice command, when the obtained analysis result is a request for a health diagnosis of the user, transmits an additional medical inquiry request to an electronic device, receives medical inquiry response information responding to the additional medical inquiry request from the electronic device, generates a prompt requiring disease diagnosis based on the health management information and the medical inquiry response information, transmits the generated prompt to a generative AI server, and can receive a diagnosis result from the generative AI server.

## Description

### [Technical Field]

The present disclosure relates to an artificial intelligence device, and more particularly, to an artificial intelligence device capable of diagnosing a user's health.

### [Background Art]

Interest in health is increasing, and in particular, as the proportion of the elderly population increases and single-person households increase, interest in health is further growing.

In particular, most people regularly perform health management activities such as taking medications, managing diet, managing exercise, and having regular medical check-ups.

In addition, there is a growing trend of various health programs such as home training apps for health management through TV or smartphones.

Conventionally, biological data collected using biometric measurement devices is analyzed through a medical AI engine to perform medical examinations such as health check-ups and disease prediction for users.

However, conventionally, health check-ups/disease prediction is performed using only biological data and does not take into account the user's personal health information, thereby having a problem in that accurate health check-ups and disease prediction for a user are not possible.

### [Disclosure]

### [Technical Problem]

The present disclosure has an object of generating a prompt for accurately identifying a user's disease to be input into a generative AI model.

The present disclosure has an object of automatically providing a hospital reservation service according to a diagnosis result output by a generative AI model.

### [Technical Solution]

An artificial intelligence device according to an embodiment of the present disclosure may include: a memory configured to store health management information of a user; a communication unit configured to communicate with an electronic device or a generative artificial intelligence (AI) server; and a processor configured to receive a voice command uttered by a user from the electronic device, obtain an analysis result of the received voice command, when the obtained analysis result is a request for a health diagnosis of the user, transmit an additional medical inquiry request to the electronic device through the communication unit, receive medical inquiry response information responding to the additional medical inquiry request from the electronic device through the communication unit, generate a prompt requiring disease diagnosis based on the health management information and the medical inquiry response information, transmit the generated prompt to the generative AI server through the communication unit, and receive a diagnosis result from the generative AI server through the communication unit.

A method of operating an artificial intelligence device according to an embodiment of the present disclosure may include: receiving a voice command uttered by a user from an electronic device; obtaining an analysis result of the received voice command; when the obtained analysis result is a request for a health diagnosis of the user, transmitting an additional medical inquiry request to the electronic device; receiving medical inquiry response information responding to the additional medical inquiry request from the electronic device; generating a prompt requiring disease diagnosis based on health management information and the medical inquiry response information; transmitting the generated prompt to a generative AI server; and receiving a diagnosis result from the generative AI server.

### [Advantageous Effects]

According to an embodiment of the present disclosure, as a prompt is generated using medical inquiry response information such as additional utterances, responses to a medical questionnaire, or results of performing a specific action of a user, an accurate diagnosis result for the user's symptoms can be provided to the user.

In addition, according to an embodiment of the present disclosure, as a reservation service for automatically reserving a hospital of the medical department for the user's disease is provided, the convenience of the user's health management can be greatly improved.

### [Description of Drawings]

FIG. 1 is a block diagram illustrating a configuration of a display device according to an embodiment of the present disclosure.
FIG. 2 is a block diagram of a remote control device according to an embodiment of the present disclosure.
FIG. 3 is a view illustrating an actual configuration example of a remote control device according to an embodiment of the present disclosure.
FIG. 4 is a view illustrating an example of utilizing a remote control device according to an embodiment of the present disclosure.
FIG. 5 is a view illustrating an artificial intelligence (AI) server according to an embodiment of the present disclosure.
FIG. 6 is a ladder diagram for explaining an operating method of an AI system according to an embodiment of the present disclosure.
FIG. 7 is a view illustrating an example of a medical questionnaire according to an embodiment of the present disclosure.
FIG. 8 is a view for explaining a process of identifying a user's symptoms and providing a remote medical service according to an embodiment of the present disclosure.
FIG. 9 is a view for explaining a process of collecting health management information of a user according to an embodiment of the present disclosure.
FIG. 10 is a view illustrating an example of outputting a generative response result according to an embodiment of the present disclosure.
FIGS. 11A and 11B are views for explaining an example of providing a hospital reservation service for treatment of a disease included in a generative response result according to an embodiment of the present disclosure.

### [Mode for Invention]

Hereinafter, embodiments relating to the present disclosure will be described in detail with reference to the accompanying drawings. The suffixes "module" and "unit" for components used in the description below are assigned or mixed in consideration of easiness in writing the specification and do not have distinctive meanings or roles by themselves.

A display device according to an embodiment of the present disclosure is an intelligent display device that adds a computer-assisted function to, for example, a broadcast reception function, and while remaining faithful to the broadcast reception function, adds an Internet function or the like and may have a more convenient interface such as a manual input device, a touch screen, or a space remote control. In addition, with the support of wired or wireless Internet functions, it is possible to connect to the Internet and a computer, and perform functions such as email, web browsing, banking, or games. A standardized general-purpose OS may be used for these various functions.

Accordingly, the display device described in the present disclosure may perform various user-friendly functions, for example, since various applications may be freely added or deleted on a general-purpose OS kernel. More specifically, the display device may be, for example, a network TV, an HBBTV, a smart TV, an LED TV, an OLED TV, or the like and in some cases, may also be applied to a smartphone.

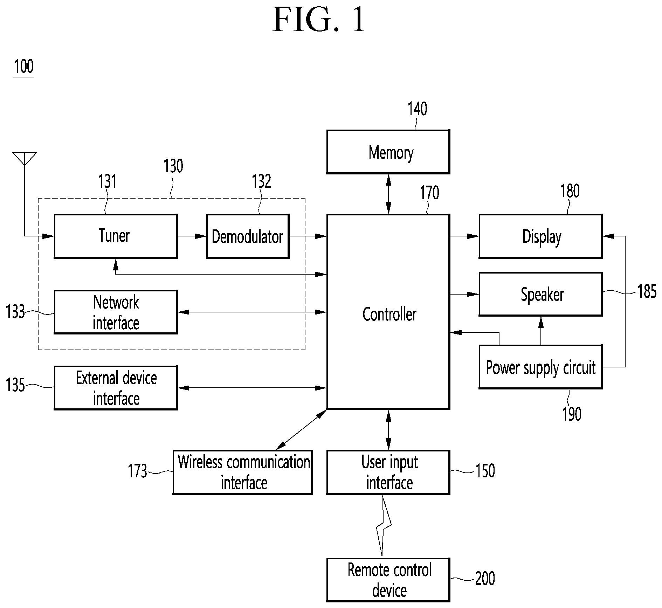

FIG. 1 is a block diagram illustrating a configuration of a display device according to an embodiment of the present disclosure.

Referring to FIG. 1, a display device 100 may include a broadcast reception part 130, an external device interface 135, a memory 140, a user input interface 150, a controller 170, a wireless communication interface 173, a display 180, a speaker 185, and a power supply circuit 190.

The broadcast reception part 130 may include a tuner 131, a demodulator 132, and a network interface 133.

The tuner 131 may select a specific broadcast channel according to a channel selection command. The tuner 131 may receive broadcast signals for the selected specific broadcast channel.

The demodulator 132 may divide the received broadcast signals into image signals, audio signals, and broadcast program-related data signals, and may restore the divided image signals, audio signals, and data signals into an output available form.

The external device interface 135 may receive an application or an application list from an adjacent external device and deliver the application or the application list to the controller 170 or the memory 140.

The external device interface 135 may provide a connection path between the display device 100 and an external device. The external device interface 135 may receive at least one of an image or audio outputted from an external device that is wirelessly or wiredly connected to the display device 100 and deliver the received image or the audio to the controller 170. The external device interface 135 may include a plurality of external input terminals. The plurality of external input terminals may include an RGB terminal, at least one High Definition Multimedia Interface (HDMI) terminal, and a component terminal.

An image signal of an external device inputted through the external device interface 135 may be outputted through the display 180. A sound signal of an external device inputted through the external device interface 135 may be outputted through the speaker 185.

An external device connectable to the external device interface 135 may be one of a settop box, a Blu-ray player, a DVD player, a game console, a sound bar, a smartphone, a PC, a USB memory, and a home theater system, but this is just exemplary.

The network interface 133 may provide an interface for connecting the display device 100 to a wired/wireless network including internet network. The network interface 133 may transmit or receive data to or from another user or another electronic device through an accessed network or another network linked to the accessed network.

Additionally, some content data stored in the display device 100 may be transmitted to a user or an electronic device, which is selected from other users or other electronic devices pre-registered in the display device 100.

The network interface 133 may access a predetermined webpage through an accessed network or another network linked to the accessed network. In other words, the network interface 133 may transmit or receive data to or from a corresponding server by accessing a predetermined webpage through the network.

The network interface 133 may receive content or data provided from a content provider or a network operator. In other words, the network interface 133 may receive content, such as movies, advertisements, games, VODs, and broadcast signals, which are provided from the content provider or the network operator, and information relating thereto through the network.

In addition, the network interface 133 may receive firmware update information and update files provided from the network operator, and may transmit data to the Internet or content provider or the network operator.

The network interface 133 may select and receive a desired application among applications open to the air through network.

The memory 140 may store signal-processed image, voice, or data signals stored by a program in order for each signal processing and control in the controller 170.

In addition, the memory 140 may perform a function for temporarily storing image, voice, or data signals output from the external device interface 135 or the network interface 133, and may store information on a predetermined image through a channel memory function.

The memory 140 may store an application or an application list input from the external device interface 135 or the network interface 133.

The display device 100 may play content files (e.g., video files, still image files, music files, document files, application files, etc.) stored in the memory 140, and may provide the content files to a user.

The user input interface 150 may transmit signals input by a user to the controller 170, or may transmit signals from the controller 170 to a user. For example, the user input interface 150 may receive or process control signals such as power on/off, channel selection, and screen setting from the remote control device 200 or transmit control signals from the controller 170 to the remote control device 200 according to various communication methods such as Bluetooth, Ultra Wideband (UWB), ZigBee, Radio Frequency (RF), and IR communication methods.

In addition, the user input interface 150 may transmit, to the controller 170, control signals input from local keys (not illustrated) such as a power key, a channel key, a volume key, and a setting key.

Image signals that are image-processed by the controller 170 may be input to the display 180 and displayed as images corresponding to the image signals. In addition, image signals that are image-processed by the controller 170 may be input to an external output device through the external device interface 135.

Voice signals processed by the controller 170 may be output to the speaker 185. In addition, voice signals processed by the controller 170 may be input to the external output device through the external device interface 135.

Additionally, the controller 170 may control overall operations of the display device 100.

In addition, the controller 170 may control the display device 100 by a user command or an internal program input through the user input interface 150, and may access the network to download a desired application or application list into the display device 100.

The controller 170 may output channel information selected by a user together with the processed image or voice signals through the display 180 or the speaker 185.

In addition, the controller 170 may output image signals or voice signals of an external device such as a camera or a camcorder, which are input through the external device interface 135, through the display 180 or the speaker 185, according to an external device image playback command received through the user input interface 150.

Moreover, the controller 170 may control the display 180 to display images, and may control the display 180 to display broadcast images input through the tuner 131, external input images input through the external device interface 135, images input through the network interface, or images stored in the memory 140. In this case, an image displayed on the display 180 may be a still image or video and also may be a 2D image or a 3D image.

Additionally, the controller 170 may play content stored in the display device 100, received broadcast content, and external input content input from the outside, and the content may be in various formats such as broadcast images, external input images, audio files, still images, accessed web screens, and document files.

Moreover, the wireless communication interface 173 may perform wired or wireless communication with an external device. The wireless communication interface 173 may perform short-range communication with an external device. For this, the wireless communication interface 173 may support short-range communication by using at least one of Bluetooth^{™}, Radio Frequency Identification (RFID), Infrared Data Association (IrDA), Ultra Wideband (UWB), ZigBee, Near Field Communication (NFC), Wireless-Fidelity (Wi-Fi), Wi-Fi Direct, and Wireless Universal Serial Bus (Wireless USB) technologies. The wireless communication interface 173 may support wireless communication between the display device 100 and a wireless communication system, between the display device 100 and another display device 100, or between networks including the display device 100 and another display device 100 (or an external server) through Wireless Area Networks. The Wireless Area Networks may be Wireless Personal Area Networks.

Herein, the other display device 100 may be a mobile terminal such as a wearable device (for example, a smartwatch, a smart glass, and a head mounted display (HMD)) or a smartphone, which is capable of exchanging data (or inter-working) with the display device 100 according to the present disclosure. The wireless communication interface 173 may detect (or recognize) a wearable device capable of communication around the display device 100.

Furthermore, if the detected wearable device is a device authenticated to communicate with the display device 100 according to the present disclosure, the controller 170 may transmit at least part of data processed in the display device 100 to the wearable device through the wireless communication interface 173. Therefore, a user of the wearable device may use the data processed by the display device 100 through the wearable device.

The display 180 may convert image signals, data signals, or OSD signals processed in the controller 170, or image signals or data signals received from the external device interface 135 into R, G, and B signals respectively to generate driving signals.

Furthermore, the display device 100 illustrated in FIG. 1 is just one embodiment of the present disclosure and thus, some of the components illustrated may be integrated, added, or omitted according to the specification of the actually implemented display device 100.

In other words, if necessary, two or more components may be integrated into one component, or one component may be divided into two or more components. Additionally, a function performed by each block is to describe an embodiment of the present disclosure and its specific operation or device does not limit the scope of the present disclosure.

According to another embodiment of the present disclosure, unlike FIG. 1, the display device 100 may receive images through the network interface 133 or the external device interface 135 and play them without including the tuner 131 and the demodulator 132.

For example, the display device 100 may be divided into an image processing device such as a set-top box for receiving broadcast signals or contents according to various network services and a content playback device for playing content input from the image processing device.

In this case, an operating method of a display device according to an embodiment of the present disclosure described below may be performed by one of the display device 100 described with reference to FIG. 1, an image processing device such as the separated set-top box, or a content playback device including the display 180 and the speaker 185.

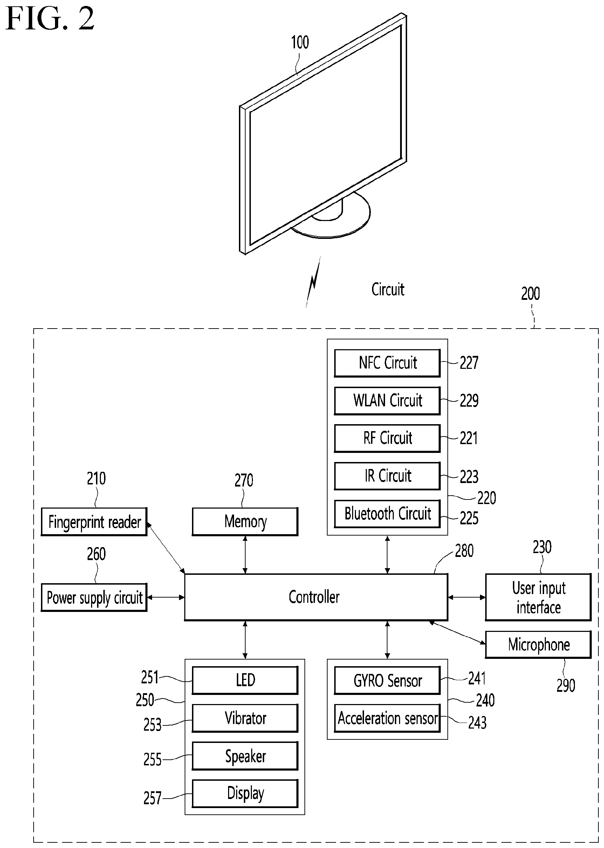

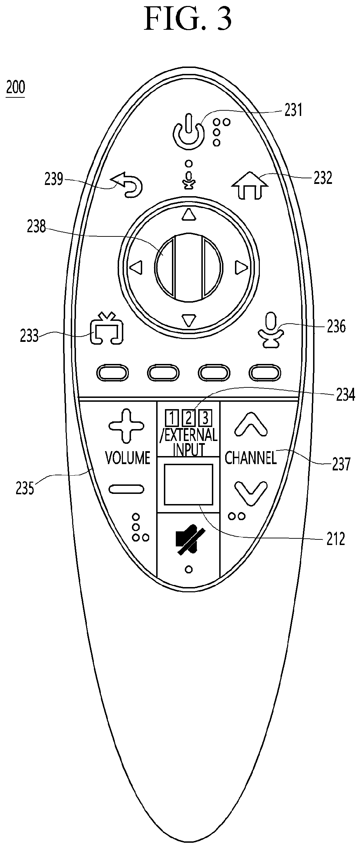

Next, a remote control device according to an embodiment of the present disclosure will be described with reference to FIGS. 2 and 3.

FIG. 2 is a block diagram of a remote control device according to an embodiment of the present disclosure, and FIG. 3 is a view illustrating an actual configuration example of the remote control device 200 according to an embodiment of the present disclosure.

First, referring to FIG. 2, a remote control device 200 may include a fingerprint recognition device 210, a wireless communication circuit 220, a user input interface 230, a sensor 240, an output interface 250, a power supply circuit 260, a memory 270, a controller 280, and a microphone 290.

Referring to FIG. 2, the wireless communication circuit 220 transmits/receives signals to/from any one of display devices according to the above-mentioned embodiments of the present disclosure.

The remote control device 200 may include a radio frequency (RF) circuit 221 capable of transmitting or receiving signals to or from the display device 100 according to an RF communication standard, and an IR circuit 223 capable of transmitting or receiving signals to or from the display device 100 according to an IR communication standard. In addition, the remote control device 200 may include a Bluetooth circuit 225 capable of transmitting or receiving signals to or from the display device 100 according to a Bluetooth communication standard. In addition, the remote control device 200 may include an NFC circuit 227 capable of transmitting or receiving signals to or from the display device 100 according to a Near Field Communication (NFC) communication standard, and a wireless LAN (WLAN) circuit 229 capable of transmitting or receiving signals to or from the display device 100 according to a WLAN communication standard.

In addition, the remote control device 200 may transmit signals containing information on the movement of the remote control device 200 to the display device 100 through the wireless communication circuit 220.

Moreover, the remote control device 200 may receive signals transmitted from the display device 100 through the RF circuit 221 and if necessary, may transmit a command for power on/off, channel change, and volume change to the display device 100 through the IR circuit 223.

The user input interface 230 may be configured with a keypad, a button, a touch pad, or a touch screen. A user may operate the user input interface 230 to input a command relating to the display device 100 to the remote control device 200. If the user input interface 230 includes a hard key button, a user may input a command relating to the display device 100 to the remote control device 200 through the push operation of the hard key button. This will be described with reference to FIG. 3.

Referring to FIG. 3, the remote control device 200 may include a plurality of buttons. The plurality of buttons may include a fingerprint recognition button 212, a power button 231, a home button 232, a live button 233, an external input button 234, a volume control button 235, a voice recognition button 236, a channel change button 237, an OK button 238, and a back button 239.

The fingerprint recognition button 212 may be a button for recognizing a user's fingerprint. According to an embodiment, the fingerprint recognition button 212 may be capable of a push operation and may receive a push operation and a fingerprint recognition operation.

The power button 231 may be a button for turning on/off the power of the display device 100.

The home button 232 may be a button for moving to the home screen of the display device 100.

The live button 233 may be a button for displaying live broadcast programs.

The external input button 234 may be a button for receiving an external input connected to the display device 100.

The volume control button 235 may be a button for controlling a volume output from the display device 100.

The voice recognition button 236 may be a button for receiving a user's voice and recognizing the received voice.

The channel change button 237 may be a button for receiving broadcast signals of a specific broadcast channel.

The OK button 238 may be a button for selecting a specific function, and the back button 239 may be a button for returning to a previous screen.

FIG. 2 is described again.

If the user input interface 230 includes a touch screen, a user may touch a soft key of the touch screen to input a command relating to the display device 100 to the remote control device 200. In addition, the user input interface 230 may include various kinds of input interfaces operable by a user, for example, a scroll key and a jog key, and this embodiment does not limit the scope of the present disclosure.

The sensor 240 may include a gyro sensor 241 or an acceleration sensor 243. The gyro sensor 241 may sense information on the movement of the remote control device 200.

For example, the gyro sensor 241 may sense information on an operation of the remote control device 200 on the basis of x, y, and z axes and the acceleration sensor 243 may sense information on a movement speed of the remote control device 200. Moreover, the remote control device 200 may further include a distance measurement sensor that senses a distance with respect to the display 180 of the display device 100.

The output interface 250 may output image or voice signals in response to the operation of the user input interface 230, or may output image or voice signals corresponding to signals transmitted from the display device 100.

A user may recognize whether the user input interface 230 is operated or the display device 100 is controlled through the output interface 250.

For example, the output interface 250 may include an LED 251 for flashing, a vibrator 253 for generating vibration, a speaker 255 for outputting sound, or a display 257 for outputting an image, if the user input interface 230 is manipulated or signals are transmitted/received to/from the display device 100 through the wireless communication interface 220.

Additionally, the power supply circuit 260 supplies power to the remote control device 200 and if the remote control device 200 does not move for a predetermined time, stops the power supply, so that power waste may be reduced.

The power supply circuit 260 may resume the supply of power if a predetermined key provided at the remote control device 200 is operated.

The memory 270 may store various kinds of programs and application data required to control or operate the remote control device 200.

If the remote control device 200 transmits/receives signals wirelessly through the display device 100 and the RF circuit 221, the remote control device 200 and the display device 100 transmit/receive signals through a predetermined frequency band.

The controller 280 of the remote control device 200 may store, in the memory 270, information on a frequency band for transmitting/receiving signals to/from the display device 100 paired with the remote control device 200 and refer to it.

The controller 280 controls general matters relating to the control of the remote control device 200. The controller 280 may transmit a signal corresponding to a predetermined key operation of the user input interface 230 or a signal corresponding to the movement of the remote control device 200 sensed by the sensor 240 to the display device 100 through the wireless communication interface 220.

In addition, the microphone 290 of the remote control device 200 may acquire voice.

A plurality of microphones 290 may be provided.

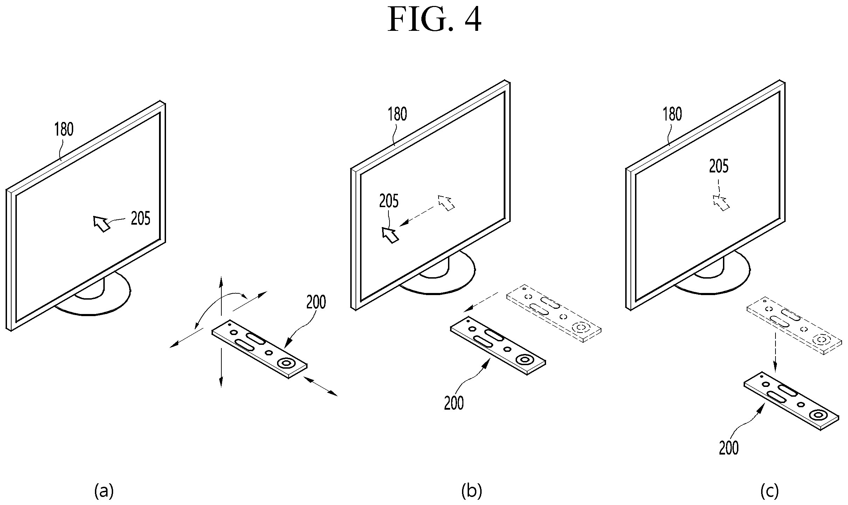

Next, FIG. 4 is described.

FIG. 4 is a view illustrating an example of utilizing a remote control device according to an embodiment of the present disclosure.

FIG. 4(a) illustrates that a pointer 205 corresponding to the remote control device 200 is displayed on the display 180.

A user may move or rotate the remote control device 200 vertically or horizontally. The pointer 205 displayed on the display 180 of the display device 100 corresponds to a movement of the remote control device 200. Since the corresponding pointer 205 is moved and displayed according to a movement on a 3D space as shown in the drawing, the remote control device 200 may be referred to as a spatial remote control device.

FIG. 4(b) illustrates that if a user moves the remote control device 200 to the left, the pointer 205 displayed on the display 180 of the display device 100 is also moved to the left accordingly.

Information on a movement of the remote control device 200 detected through a sensor of the remote control device 200 is transmitted to the display device 100. The display device 100 may calculate the coordinates of the pointer 205 from the information on the movement of the remote control device 200. The display device 100 may display the pointer 205 to match the calculated coordinates.

FIG. 4(c) illustrates that while a specific button in the remote control device 200 is pressed, a user moves the remote control device 200 away from the display 180. Thus, a selected region in the display 180 corresponding to the pointer 205 may be zoomed in and displayed in an enlarged size.

Conversely, if a user moves the remote control device 200 closer to the display 180, a selected region in the display 180 corresponding to the pointer 205 may be zoomed out and displayed in a reduced size.

On the other hand, when the remote control device 200 is moved away from the display 180, the selected region may be zoomed out, and when the remote control device 200 is moved closer to the display 180, the selected region may be zoomed in.

Additionally, when a specific button in the remote control device 200 is pressed, recognition of up, down, left, or right movement may be excluded. In other words, when the remote control device 200 is moved away from or closer to the display 180, the up, down, left, or right movement may not be recognized and only the back and forth movement may be recognized. When a specific button in the remote control device 200 is not pressed, only the pointer 205 is moved according to the up, down, left, or right movement of the remote control device 200.

Moreover, the moving speed or moving direction of the pointer 205 may correspond to the moving speed or moving direction of the remote control device 200.

Furthermore, a pointer in this specification means an object displayed on the display 180 in response to an operation of the remote control device 200. Therefore, in addition to the arrow form displayed as the pointer 205 in the drawing, various forms of objects are possible. For example, the above concept may include a point, a cursor, a prompt, and a thick outline. In addition, the pointer 205 may be displayed in correspondence to one point of a horizontal axis or a vertical axis on the display 180 and also may be displayed in correspondence to a plurality of points such as a line and a surface.

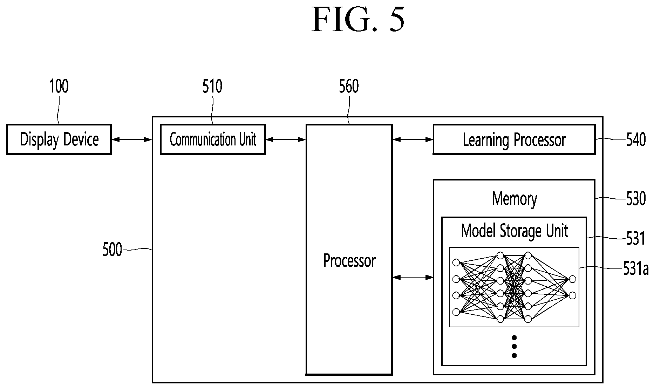

FIG. 5 is a view illustrating an artificial intelligence (AI) server according to an embodiment of the present disclosure.

Referring to FIG. 5, the AI server 500 may refer to a device that trains an artificial neural network using a machine learning algorithm or uses a trained artificial neural network.

The AI server 500 may be configured with a plurality of servers to perform distributed processing, or may be defined as a 5G network.

The AI server 500 may be included as part of a configuration of the AI device 100 and may perform at least part of AI processing together.

The AI server 500 may include a communication unit 510, a memory 530, a learning processor 540, and a processor 560.

The communication unit 510 may transmit or receive data to or from an external device such as the display device 100.

The memory 530 may include a model storage unit 531.

The model storage unit 531 may store a model (or artificial neural network, 531a) that is being trained or has been trained through the learning processor 540.

The learning processor 540 may train the artificial neural network 531a using training data. The trained model may be used while being mounted on the AI server 500 of the artificial neural network, or may be mounted on and used in an external device such as the display device 100.

The trained model may be implemented in hardware, software, or a combination of hardware and software. When part or all of the trained model is implemented in software, one or more instructions constituting the trained model may be stored in the memory 530.

The processor 560 may infer a result value for new input data using the trained model and may generate a response or control command based on the inferred result value.

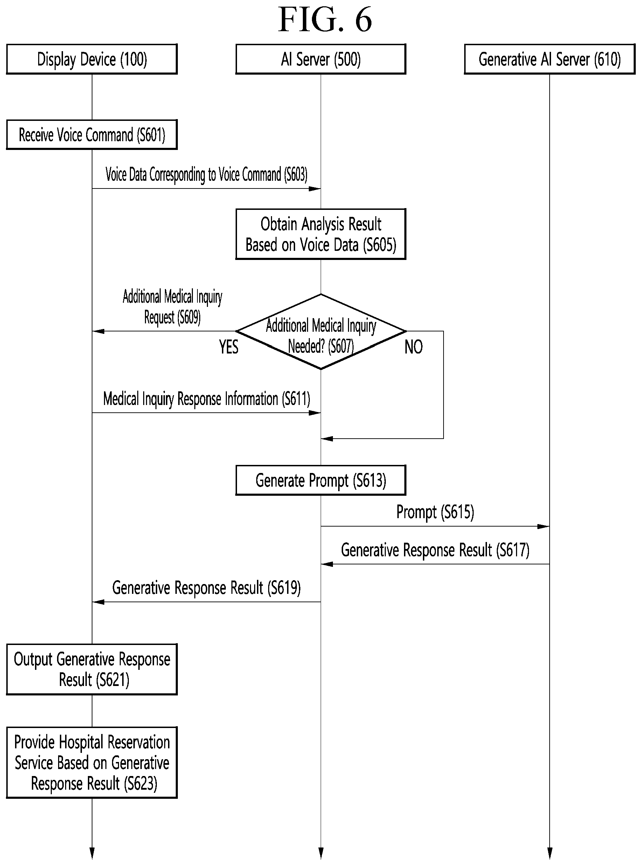

FIG. 6 is a ladder diagram for explaining an operating method of an AI system according to an embodiment of the present disclosure.

Referring to FIG. 6, the AI system may include the display device 100, an AI server 500, and a generative AI server 610.

The AI server 500 may be a Natural Language Processing (NLP) server.

The AI server 500 may be referred to as an artificial intelligence device.

The controller 170 of the display device 100 may receive a voice command (S601).

In one embodiment, the controller 170 may receive a voice command uttered by a user through a microphone (not illustrated).

The controller 170 may receive a voice command from the remote control device 200. The remote control device 200 may receive a voice command uttered by a user and transmit it to the display device 100.

**The controller 170 of the display device 100 may transmit voice data corresponding to the voice command to the AI server 500 through the network interface 133 (S603).**

In one embodiment, the controller 170 may transmit voice data together with user identification information to the AI server 500 through the network interface 133.

**The processor 560 of the AI server 500 may obtain an analysis result of the voice command based on the received voice data (S605).**

In one embodiment, the processor 560 may convert voice data into text data using a Speech To Text (STT) engine stored in the memory 530.

In another embodiment, the processor 560 may transmit voice data to an STT server and receive text data converted from the STT server.

The processor 560 may obtain an analysis result of the text data using a Natural Language Processing (NLP) engine stored in the memory 530.

The analysis result may include an analysis result of the intent of the voice command.

For example, when the voice command is <I am sick>, the analysis result may indicate that the intent is to diagnose the user's health. That is, the analysis result of the voice command may indicate an intent to request a diagnosis of a disease or occurrence of a disease.

**The processor 560 of the AI server 500 may determine whether additional medical inquiry is necessary based on the analysis result of the voice command (S607).**

In one embodiment, when the analysis result does not include the user's symptoms, the processor 560 may determine that additional medical inquiry is necessary.

In another embodiment, even when the analysis result includes the user's symptoms, when additional diagnostic information is needed to identify the disease, the processor 560 may determine that additional medical inquiry is necessary.

**When the processor 560 of the AI server 500 determines that additional medical inquiry is necessary, the processor 560 may transmit an additional medical inquiry request to the display device 100 through the communication unit 510 (S609).**

The additional medical inquiry request may include one or more of a medical questionnaire, a request for additional utterance by the user, or a request for a result of performing a specific action.

In one embodiment, the additional medical inquiry request may include a medical questionnaire previously stored in a health management database. The health management database may be the memory 530 of the AI server 500, or may be provided separately from the AI server 500.

In one embodiment, the medical questionnaire may be obtained from the generative AI server 600. When the processor 560 determines that additional medical inquiry is necessary, the processor 560 may request a medical questionnaire from the generative AI server 600 and receive the medical questionnaire from the generative AI server 600.

When the processor 560 already has answers stored for some of the plurality of inquiries provided in the medical questionnaire, the processor 560 may include only the remaining inquiries in the medical questionnaire.

In another embodiment, the additional medical inquiry request may be a request to inquire about one or more of the type of symptom or the area where the symptom is present.

The processor 560 may receive a user's response to an initial medical inquiry before step S601.

When the processor 560 determines that additional medical inquiry is necessary based on the received user's response, the processor 560 may generate an additional medical inquiry request and transmit it to the display device 100.

The additional medical inquiry request may include one or more of a medical questionnaire, a request for additional utterance by the user, or a request for a result of performing a specific action.

The communication unit 510 may be referred to as a communication interface. The communication unit 510 may include a circuit for Internet communication.

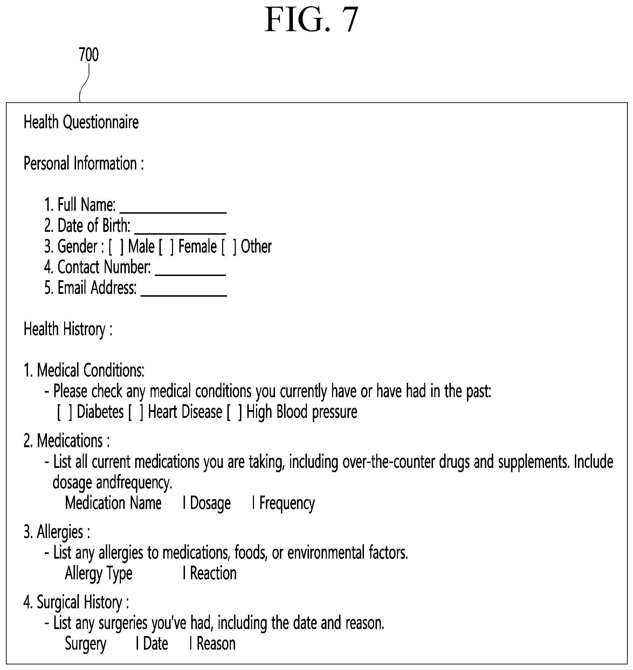

FIG. 7 is a view illustrating an example of a medical questionnaire according to an embodiment of the present disclosure.

The AI server 500 may transmit a medical questionnaire 700 to the display device 100. The medical questionnaire 700 may be one generated by the generative AI server 600.

The medical questionnaire 700 may be a form that inquires about the user's personal information, diseases, allergies, surgical history, type of symptoms, and area of symptoms.

The display device 100 may receive responses to the inquiries of the medical questionnaire 700.

Referring back to FIG. 6.

**The controller 170 of the display device 100 may obtain medical inquiry response information in response to the additional medical inquiry request and may transmit the obtained medical inquiry response information to the AI server 500 through the network interface 133 (S611).**

In one embodiment, the medical inquiry response information may include responses to the medical questionnaire. In this case, the controller 170 may receive responses to the medical questionnaire through text input or utterance input.

In another embodiment, when the additional medical inquiry request is a request to inquire about one or more of the type of symptom or the area where the symptom is present, the controller 170 may output a notification to input the type of symptom, the area where the symptom is present, or a notification to input a result of performing a specific action.

The controller 170 may receive one or more of the type of symptom or the area where the symptom is present through the user input interface 150. The controller 170 may receive information on an additional voice command additionally uttered by the user or a result of a specific action performed by the user, and may transmit information on the received additional voice command or the specific action to the AI server 500 through the network interface 133.

For example, the information on the result of performing a specific action may be one or more of a measured value of blood glucose or a measured value of blood pressure.

**The processor 560 of the AI server 500 may generate a prompt for a response of the generative AI based on the user's health management information and medical inquiry response information (S613).**

In one embodiment, the processor 560 may obtain the user's health management information from a customer health management database. The user's health management information may include one or more of user identification information, the user's age, disease management items, and information on medications being taken.

The customer health management database may be included in the AI server 500 or may be provided separately from the AI server 500.

The processor 560 may generate a prompt for obtaining a response result of a generative AI model provided in the generative AI server based on the user's health management information and medical inquiry response information.

In one embodiment, the processor 560 may extract keywords from each of the user's health management information and medical inquiry response information, and may generate one sentence by combining the extracted keywords. The processor 560 may obtain the generated single sentence as a prompt.

In one embodiment, the processor 560 may extract keywords using any one of text rank and topic modeling techniques.

Text rank is a method of extracting keywords using a graph-based algorithm, which may be a method of representing sentences or words as graph nodes and identifying important words by analyzing the connection relationships between nodes.

Topic modeling technique may be a method of identifying main topics in text data and extracting keywords related to the identified topics.

The processor 560 may generate a prompt requiring disease diagnosis based on the health management information and medical inquiry response information.

The prompt may include combined keywords and a command requesting information related to a disease.

For example, the prompt may be <I have a history of hypertension, my blood glucose level is 135, please research diseases related to this>.

The history of hypertension may be the user's health management information, and the blood glucose level may be the medical inquiry response information.

In another embodiment, the processor 560 may generate a prompt based on the user's health management information, medical inquiry response information, and medical information stored in a medical database. The medical database may be included in the AI server 500 or may be provided separately from the AI server 500.

The medical database may include medical information. The medical information may include one or more of information on medical papers, medical terminology, clinical trial information, and medical statistics information. The information on medicine may include professional medical knowledge.

The processor 560 may extract related medical terminology from the medical database based on one or more of the user's health management information or medical inquiry response information.

For example, the prompt may be <I have a history of hypertension, my blood glucose level is 135, please research diseases related to insulin>.

The history of hypertension may be the user's health management information, the blood glucose level may be the medical inquiry response information, and insulin may be a medical term extracted from the medical database related to the blood glucose level.

The processor 560 may generate a prompt based on the user's health management information, medical inquiry response information, and medical information. When medical terminology is used in the prompt, a more accurate disease of the user can be predicted.

In one embodiment, the processor 560 may generate a prompt each time the user utters a voice command, or may use a pre-generated prompt in a fixed format.

The processor 560 may determine whether to use the pre-generated prompt in a fixed format based on the analysis result of the user's voice command.

The processor 560 may store a plurality of pre-generated prompts in the memory 530.

For example, the pre-generated prompt may have the following fixed formats.

That is, the prompt may have a fixed format such as <user's past disease history + measured biometric index value + related disease search> or <user's current disease + measured biometric index value + related disease search>.

When the analysis result of the user's voice command is disease diagnosis, the processor 560 may extract the pre-generated prompt from the memory 530 and transmit the extracted prompt to the generative AI server 610.

In this way, by using pre-generated prompts according to the analysis result of the voice command, the amount of computation and time required for generating prompts can be reduced.

**The processor 560 of the AI server 500 may transmit the prompt to the generative AI server 610 through the communication unit 510 (S615), and may receive a generative response result from the generative AI server 610 (S617).**

The generative AI server 610 may include one or more generative AI models.

The generative AI model may be an artificial intelligence-based model that uses deep learning or machine learning to output a response to a prompt.

The generative AI model may be a Large Language Model (LLM) trained to understand human language and generate responses according to input.

The Large Language Model may be referred to as a natural language generative AI model.

The natural language generative AI model may be a model trained using deep learning architecture such as a recurrent neural network (RNN) and a transformer model using an attention mechanism through large-scale text data.

Data for training the natural language generative AI model may be collected from the web or various text sources.

The natural language generative AI model may output generative response information using the received prompt as input.

The generative response result may include a diagnosis result for the user's health.

The generative response result may include one or more of the name of the disease, temporary measures, medical department, and hospital information for treating the disease.

**The processor 560 of the AI server 500 may transmit the generative response result to the display device 100 through the communication unit 510 (S619).**

**The controller 170 of the display device 100 may output the generative response result (S621).**

The controller 170 may display the generative response result on the display 180.

**The controller 170 of the display device 100 may provide a hospital reservation service based on the generative response result (S623).**

The controller 170 may determine the medical department included in the generative response result and reserve a hospital having the medical department.

The controller 170 may reserve a hospital having the medical department based on the user's location.

The controller 170 may search for a hospital having the medical department within a certain distance from the user's location and reserve the searched hospital.

The controller 170 may display hospital reservation information on the display 180 according to the hospital reservation, or may transmit hospital reservation information to the user's mobile terminal through the wireless communication interface 173.

The hospital reservation information may include the reservation date and reservation time of the hospital.

The controller 170 may output a notification for the hospital reservation information before the reservation date and reservation time of the hospital arrive.

In this way, according to an embodiment of the present disclosure, as a prompt is generated using medical inquiry response information such as additional utterances, responses to a medical questionnaire, or results of performing a specific action of a user, an accurate diagnosis result for the user's symptoms can be provided to the user.

In addition, according to an embodiment of the present disclosure, as a reservation service for automatically reserving a hospital of the medical department for the user's disease is provided, the convenience of the user's health management can be greatly improved.

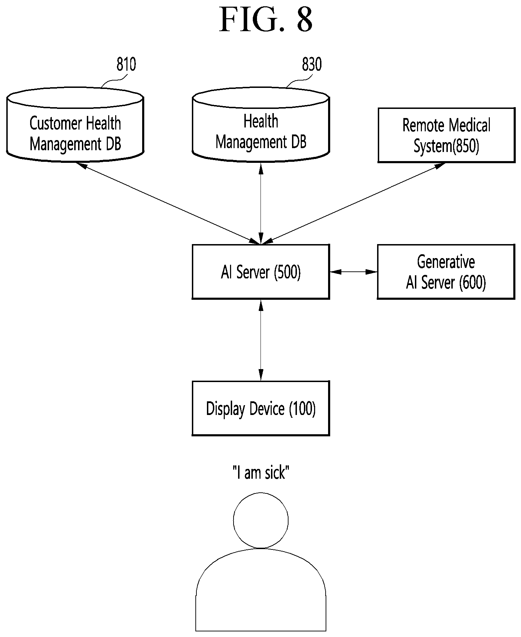

FIG. 8 is a view for explaining a process of identifying a user's symptoms and providing a remote medical service according to an embodiment of the present disclosure.

Referring to FIG. 8, a customer health management DB 810 may store health management information of each of a plurality of users.

The display device 100 may collect the user's health management information and transmit the collected user's health management information to the AI server 500. The AI server 500 may match the user's health management information with user identification information and store it in the customer health management DB 810.

The process of collecting the user's health management information will be described later.

The health management DB 830 may store health management methods corresponding to diseases, types of major diseases by age, and diet/exercise/precautions/medication information according to major diseases.

The health management DB 830 may further store a medical questionnaire. The medical questionnaire may be one generated by the generative AI server 600.

A user utters a voice command of <I am sick>. The display device 100 may obtain the voice command and transmit the obtained voice command and user identification information to the AI server 500.

The AI server 500 may analyze the intent of the voice command and obtain an analysis result.

When additional medical inquiry is needed based on the obtained analysis result, the AI server 500 may transmit an additional medical inquiry request to the display device 100.

The additional medical inquiry request may include one or more of a medical questionnaire, a request for additional utterance about symptoms, or a request for a result of performing a specific action.

The display device 100 may output the additional medical inquiry request on the display 180.

The display device 100 may receive medical inquiry response information responding to the additional medical inquiry request, and may transmit the received medical inquiry response information to the AI server 500.

The AI server 500 may store the medical inquiry response information in the customer health management DB 810 to update the user's health management information.

When additional medical inquiry request is needed based on the medical inquiry response information, the AI server 500 may again transmit an additional medical inquiry request to the display device 100.

The AI server 500 may generate a prompt requesting a health diagnosis of the user based on the user's health management information and medical inquiry response information.

The AI server 500 may transmit the generated prompt to the generative AI server 600. The generative AI server 600 may obtain a generative response result including a diagnosis result of the user's health for the prompt using a natural language generative AI model.

The diagnosis result may include the name of the disease, temporary measures, preventive measures, and hospital reservation information.

The AI server 500 may transmit the generative response result to the display device 100.

The display device 100 may display a notification window inquiring whether to use the generative response result and the remote medical service.

When the AI server 500 receives a command agreeing to use the remote medical service from the display device 100, the AI server 500 may connect the remote medical system 850 and the display device 100.

A user may receive the remote medical service through a camera or microphone.

The remote medical result may be stored in the customer health management DB 810 and used to update the user's health management information.

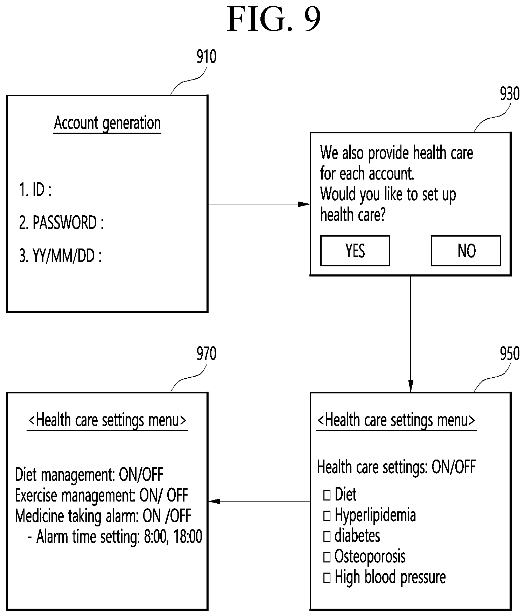

FIG. 9 is a view for explaining a process of collecting health management information of a user according to an embodiment of the present disclosure.

Referring to FIG. 9, the display device 100 may display an account creation window 910 for creating a user's account. The account creation window 910 may include input windows for collecting the user's ID, password, and date of birth.

After user information is input through the account creation window 910, the display device 100 may display a pop-up window 930 inquiring about the setting of health management care.

When the display device 100 receives input for setting health management care through the pop-up window 930, the display device 100 may display a first health management settings menu 950.

The first health management settings menu 950 may be a menu for identifying disease items that the user has.

When one of the plurality of disease items included in the first health management settings menu 950 is selected, the display device 100 may display a second health management settings menu 970.

The second health management settings menu 970 may include health management items for the selected disease item. The health management items may include a diet management item, an exercise management item, and a medication taking item.

The display device 100 may transmit the user's health management information and user identification information obtained through the first health management settings menu 950 and the second health management settings menu 970 to the AI server 500.

The user identification information may include the user's ID.

The AI server 500 may match the received user's health management information with user identification information and store it in the customer health management DB 810.

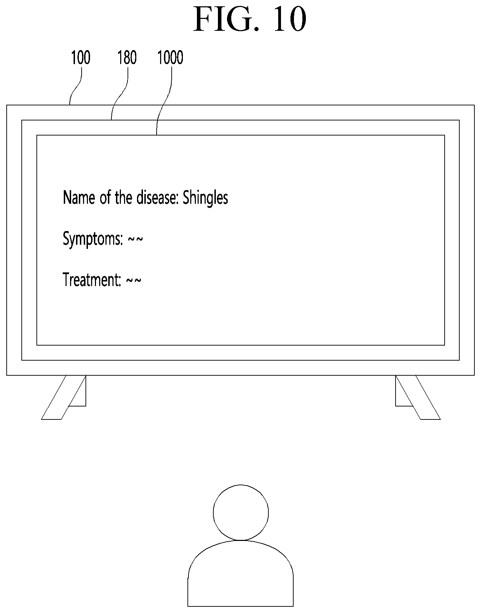

FIG. 10 is a view illustrating an example of outputting a generative response result according to an embodiment of the present disclosure.

Referring to FIG. 10, the display device 100 may display a generative response result 1000 indicating the user's health diagnosis result received from the AI server 500 on the display 180.

The generative response result 1000 may include the name of the disease, symptoms of the disease, and measures for the disease.

The generative response result 1000 is a result based on the user's health management information and medical inquiry response information, and may include a more accurate examination result compared to a diagnosis predicted using only biometric data.

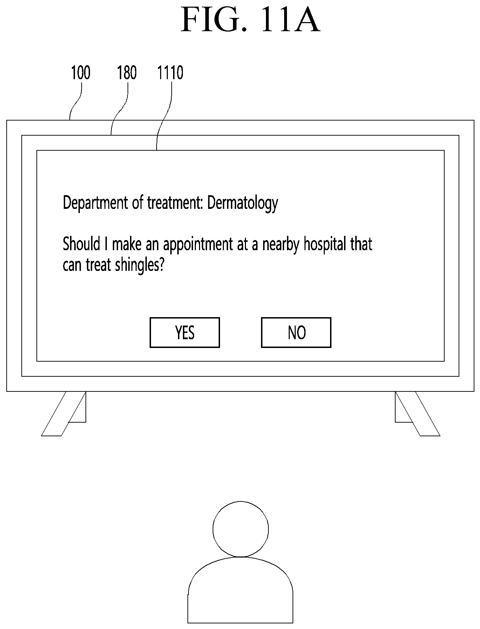

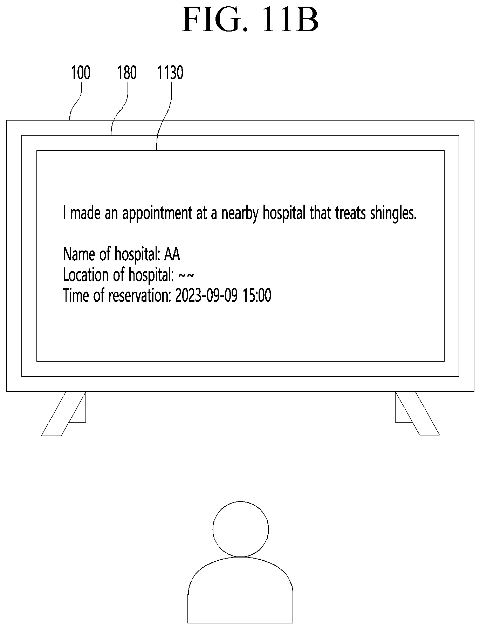

FIGS. 11A and 11B are views for explaining an example of providing a hospital reservation service for treatment of a disease included in a generative response result according to an embodiment of the present disclosure.

Referring to FIG. 11A, the display device 100 may display a hospital reservation inquiry window 1110 on the display 180 inquiring about the reservation of a hospital that treats the medical department of the disease included in the generative response result 1000 of FIG. 10.

When the display device 100 receives an agreement input through the hospital reservation inquiry window 1110, the display device 100 may reserve a hospital that treats the disease based on the user's location. The user's location may be the same as the location of the display device 100.

The display device 100 may obtain the location of the display device 100 as the user's location and may search for a hospital within a certain distance from the user's location that treats the disease of the generative response result 1000.

The display device 100 may obtain the treatment hours information of the searched hospital and may perform a reservation based on the obtained treatment hours information of the hospital.

Accordingly, the display device 100 may display hospital reservation information 1130 on the display 180.

The hospital reservation information 1130 may include the name of the hospital, the location of the hospital, and the reservation time of the hospital.

The display device 100 may output a notification including the hospital reservation information 1130 on the display 180 a certain time before the reservation date or reservation time of the hospital.

The display device 100 may transmit a notification including the hospital reservation information 1130 to a mobile terminal such as the user's smartphone through the wireless communication interface 173 a certain time before the reservation time of the hospital.

In another embodiment, instead of the display device 100, the AI server 500 may generate hospital reservation information based on the generative response result.

The AI server 500 may transmit the generated hospital reservation information to the display device 100, and the display device 100 may display the received hospital reservation information.

The AI server 500 may receive the user's location from the display device 100 and may search for a hospital that can treat the disease included in the diagnosis result adjacent to the received user's location.

The AI server 500 may search for the treatment hours and reservation time of the searched hospital and may automatically perform a medical reservation for treatment at the hospital.

When the reserved treatment time arrives, the AI server 500 may transmit a control command to the display device 100 to cause the display device 100 to output a notification of the hospital reservation time.

In this way, according to an embodiment of the present disclosure, a reservation service for a hospital for treating the disease diagnosed by the user can be automatically provided.

A user can receive disease diagnosis and hospital reservation service for the disease only with simple utterances at home, responses to a medical questionnaire, or performing a specific action, enabling rapid response to diseases and convenient hospital reservations.

According to one embodiment of the present disclosure, the above-described method can be implemented as processor-readable code on a medium on which a program is recorded. Examples of the processor-readable medium include ROM, RAM, CD-ROM, magnetic tape, floppy disk, and optical data storage devices.

The display device described above is not limited to the configurations and methods of the above-described embodiments, but the embodiments may be configured by selectively combining all or part of each embodiment so that various modifications can be made.

## Claims

1. An artificial intelligence device, comprising:
a memory configured to store health management information of a user;
a communication unit configured to communicate with an electronic device or a generative artificial intelligence (AI) server; and
a processor configured to receive a voice command uttered by a user from the electronic device, obtain an analysis result of the received voice command, when the obtained analysis result is a request for a health diagnosis of the user, transmit an additional medical inquiry request to the electronic device through the communication unit, receive medical inquiry response information responding to the additional medical inquiry request from the electronic device through the communication unit, generate a prompt requiring disease diagnosis based on the health management information and the medical inquiry response information, transmit the generated prompt to the generative AI server through the communication unit, and receive a diagnosis result from the generative AI server through the communication unit.

2. The artificial intelligence device of claim 1, wherein the processor is configured to obtain medical terminology from a medical database, and generate a prompt based on the health management information, the medical inquiry response information, and the medical terminology.

3. The artificial intelligence device of claim 2, wherein the additional medical inquiry request includes one or more of a medical questionnaire, a request for additional utterance by the user, or a request for a result of performing a specific action.

4. The artificial intelligence device of claim 1, wherein the diagnosis result includes one or more of a name of a disease, measures, medical department, or information on a hospital treating the disease.

5. The artificial intelligence device of claim 4, wherein the processor is configured to search for the hospital based on the diagnosis result, and perform a medical reservation through the searched hospital.

6. The artificial intelligence device of claim 5, wherein the processor is configured to transmit a control command to the electronic device to cause the electronic device to output the reservation information when a reservation date or a reservation time included in the reservation information arrives.

7. The artificial intelligence device of claim 1, wherein the processor is configured to extract keywords from each of the health management information and the medical inquiry response information, and generate the prompt based on the extracted keywords.

8. A method of operating an artificial intelligence device, comprising:
receiving a voice command uttered by a user from an electronic device;
obtaining an analysis result of the received voice command;
transmitting an additional medical inquiry request to the electronic device, when the obtained analysis result is a request for a health diagnosis of the user;
receiving medical inquiry response information responding to the additional medical inquiry request from the electronic device;
generating a prompt requiring disease diagnosis based on health management information and the medical inquiry response information;
transmitting the generated prompt to a generative AI server; and
receiving a diagnosis result from the generative AI server.

9. The method of operating an artificial intelligence device of claim 8, wherein generating the prompt comprises obtaining medical terminology from a medical database, and generating a prompt based on the health management information, the medical inquiry response information, and the medical terminology.

10. The method of operating an artificial intelligence device of claim 9, wherein the additional medical inquiry request includes one or more of a medical questionnaire, a request for additional utterance by the user, or a request for a result of performing a specific action.

11. The method of operating an artificial intelligence device of claim 8, wherein the diagnosis result includes one or more of a name of a disease, measures, medical department, or information on a hospital treating the disease.

12. The method of operating an artificial intelligence device of claim 11, further comprising:
searching for the hospital based on the diagnosis result; and
obtaining reservation information of the searched hospital.

13. The method of operating an artificial intelligence device of claim 12, further comprising:
transmitting a control command to the electronic device to cause the electronic device to output the reservation information when a reservation date or a reservation time included in the reservation information arrives.

14. The method of operating an artificial intelligence device of claim 8, wherein generating the prompt comprises extracting keywords from each of the health management information and the medical inquiry response information, and generating the prompt based on the extracted keywords.
